(19) Europäisches Patentamt
European Patent Office
Office européen des brevets

(11) Veröffentlichungsnummer: **0 060 911**
**B1**

(12) # EUROPÄISCHE PATENTSCHRIFT

(45) Veröffentlichungstag der Patentschrift: **19.06.85**

(51) Int. Cl.⁴: **A 61 K 6/06**

(21) Anmeldenummer: **81102198.9**

(22) Anmeldetag: **24.03.81**

(54) **Dentales Füllungsmaterial.**

(43) Veröffentlichungstag der Anmeldung: **29.09.82 Patentblatt 82/39**

(45) Bekanntmachung des Hinweises auf die Patenterteilung: **19.06.85 Patentblatt 85/25**

(84) Benannte Vertragsstaaten: **AT BE CH DE FR GB IT LI LU NL SE**

(56) Entgegenhaltungen:
**DE - A - 2 028 702**
**GB - A - 1 392 763**

(73) Patentinhaber: **Blendax-Werke R. Schneider GmbH & Co., Rheinallee 88, D-6500 Mainz (DE)**

(72) Erfinder: **Orlowski, Jan A., Dr., 1304 Rubio Street, Altadena California 91001 (US)**
Erfinder: **Butler, David V., 2825 E. Cortez Street, West Covina California 91791 (US)**

**EP 0 060 911 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein neues dentales Füllungsmaterial auf Basis polymerischer Verbindungen.

Dentale Füllungsmaterialien auf der Basis polymerisierbarer Verbindungen, sogenannte »Composites«, enthalten neben einem oder mehreren polymerisierbaren Monomeren, Polymerisationskatalysatoren, Beschleunigern, UV-Stabilisatoren, etc. obligatorisch einen, in der Regel mineralischen, Füllstoff.

Hierbei wird unterschieden zwischen sogenannten Makrofillern«, d. h. Füllstoffen mit einem mittleren Teilchendurchmesser von mehr als 0,5 Mikrometer, vorzugsweise 1 bis 2 Mikron, bis zu etwa 70, vorzugsweise etwa 50 Mikrometer. Derartige Füllstoffe sind in »Composite«-Materialien in einer Menge von etwa 60 bis maximal etwa 80 Gew.-% der Gesamtzusammensetzung enthalten. Je höher der Anteil an Füllstoff, desto besser die physikalischen Eigenschaften des auspolymerisierten Materials; je höher der Anteil an »Makrofiller«, desto schlechter allerdings auch die Polierfähigkeit der auspolymerisierten (ausgehärteten) Füllung.

Um die Polierfähigkeit solcher Füllungen zu verbessern, hat man deshalb auch bereits vorgeschlagen, Füllstoffe mit geringeren Teilchengrößen zum Einsatz in dentalen Füllungsmaterialien zu entwikkeln, die der Füllung eine gewisse Polierfähigkeit verleihen.

So beschreiben die DE-ASen Nr. 2 403 211 und 2 405 578 den Einsatz von anorganischen Füllstoffen, insbesondere Silica, mit Teilchengrößen von 5 bis 700 nm in dentalen Füllungsmaterialien.

Diese Verbesserung der Polierfähigkeit geht allerdings wiederum zu Lasten der physikalischen Eigenschaften, insbesondere der Wasserabsorption der Füllung, da diese »Mikrofiller« mit einem mittleren Teilchendurchmesser von etwa 10 bis 300 nm nur zu einem Anteil von etwa 15 bis etwa 50 Gew.-% in das dentale Füllungsmaterial einarbeitbar sind; d. h., das Verhältnis Harz zu Füllstoff im Füllungsmaterial beträgt im günstigsten Fall etwa 1 : 1.

Als »Mikrofiller« werden dabei in der Regel silanisierte poröse Siliciumdioxide eingesetzt, wobei der Silanisierungsrad maximal 2,5% Organosilicon-Gehalt beträgt.

Es wurde dehalb bereits vorgeschlagen, Gemische aus »Makrofillern«, d. h. Füllstoffen mit einer mittleren Teilchengröße von etwa 1 bis etwa 40 Mikrometer, und »Mikrofillern«, d. h. Füllstoffen mit einer mittleren Teilchengröße von etwa 10 bis etwa 300 nm, in dentalen Füllungsmaterialien einzusetzen.

So beschreibt die DE-OS 2 705 720 dentale Füllungsmaterialien, die Gemische aus 70 bis 95 Gew.-% eines Füllstoffs mit einer Teilchengröße von 0,7 bis 25 Mikrometer und 5 bis 30 Gew.-% eines Füllstoffs mit einer Teilchengröße 0,2 bis 0,7 Mikrometer enthalten.

Derartige Füllungsmaterialien weisen zwar eine verbesserte Polierbarkeit auf, jedoch ist ihre Wasserabsorption inakzeptabel hoch.

Es wurde nun gefunden, daß man einem dentalen Füllungsmaterial eine zufriedenstellende Polierbarkeit bei Beibehaltung der guten physikalischen Eigenschaften, insbesondere eines niedrigen Wasserabsorptionswertes, verleihen kann, wenn man als Füllstoff ein anorganisches Material, insbesondere poröses Siliciumdioxid, mit einem mittleren Teilchendurchmesser zwischen 5 und 300 nm einsetzt, das in einem solchen Maße silanisiert ist, daß sein Organosilikongehalt 5 bis 25, vorzugsweise 8 bis 20, insbesondere zwischen 10 bis 12 und 18% (bezogen auf den Füllstoff) beträgt.

Überraschenderweise ist es mit einem solchen Füllstoff möglich, eine Füllstoff : Harz-Relation von bis zu 6,3 : 1 zu erreichen, die weit oberhalb der bisher bekannten Werte liegt.

Ein Füllungsmaterial mit besonders eindrucksvollen physikalischen Eigenschaften wird dann erhalten, wenn man 50 bis 88 Gew.-%, bezogen auf die Gesamtzusammensetzung, eines Füllstoffgemisches aus 8, insbesondere 12, bis 60 Gew.-% mindestens eines silanisierten anorganischenn Füllstoffs, insbesondere eines Siliciumdioxids, mit einem mittleren Teilchendurchmesser von 5 bis 300 nm und einem Organosilikon-Gehalt zwischen 5 und 25%, und 40 bis 92 Gew.-% mindestens eines vorzugsweise nichtporösen Füllstoffs mit einem mittleren Teilchendurchmesser von mindestens 0,5 Mikrometer zusammen mit mindestens einem polymerisierbaren Monomeren und sonstigen, in dentalen Kunststoff-Füllungsmaterialien üblichen Bestandteilen einsetzt.

Eine mit einem solchen Material hergestellte Füllung weist optimale Eigenschaften, insbesondere hinsichtlich ihrer Polierfähigkeit, Härte, Zugfestigkeit und Wasserabsorption, auf.

Wie bereits ausgeführt, handelt es sich bei dem als »Mikrofiller« erfindungsgemäß zum Einsatz kommenden Füllstoff vorzugsweise um ein poröses Siliciumdioxid.

Die Silanisierung dieses Materials zur Erreichung des hohen Organosilikon-Gehalts kann prinzipiell mit jedem geeigneten Organosilan der allgemeinen Formel

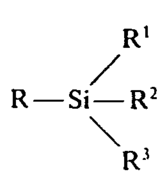

wobei R, $R^1$, $R^2$ und $R^3$ gleiche oder verschiedene organische Reste darstellen mit der Maßgabe, daß

mindestens ein Rest eine OH-Gruppe oder einen in eine OH-Gruppe, beispielsweise durch Hydrolyse überführbaren Rest, insbesondere eine Alkoxygruppe, bedeutet, erfolgen. Bevorzugte Organosilane sind (Meth)Acroylpropyldihydroxymethoxysilan, (Meth)Acroylpropylhydroxydimethoxysilan, (Meth)-Acroylpropyltrimethoxysilan oder deren Gemische; jedoch sind beispielsweise auch Vinyltriethoxysilan oder Vinyltri(methoxy)silan geeignete Silanisierungmsittel. Unter Organosilikon-Gehalt des Füllstoffs wird der Gehalt an auf den Füllstoff aufgebrachtem Organosilan verstanden.

Bei dem gegebenenfalls, gemäß einer bevorzugten Ausführungsform der Erfindung, mit dem »Mikrofiller« mit einem Organosilikon-Gehalt zwischen 5 und 25% gemeinsam zum Einsatz gelangenden »Makrofiller« kann es sich um einen beliebigen, röntgenstrahlendurchlässigen oder -undurchlässigen, für diesen Zweck vorgeschlagenen Füllstoff handeln, der gegebennenfalls ebenfalls in an sich bekannter Weise silanisiert sein kann, wobei der maximale Organosilikon-Gehalt hierbei etwa 2,5%, bezogen auf den Füllstoff, beträgt.

Solche geeigneten Füllstoffe sind die verschiedenen Siliciumdioxid-Modifikationen wie Glas in Form von pulverisiertem Glas, Glasfasern, Quarzit, Christobalit etc. für röntgendurchlässige Füllstoffe und Bariumaluminiumsilikat, Bariumaluminiumboratsilikat, Lithiumaluminiumsilikat oder Glaskeramik-Füllstoffe für röntgenundurchlässige Füllungsmaterialien. Geeignete röntgenundurchlässige Füllstoffe sind beispielsweise in den US-PSen 3 801 344, 3 808 170 und 3 975 203 sowie der DE-OS 2 347 591 beschrieben.

Eine Zusammenfassung geeigneter Füllungsmaterialien findet sich bei R. L. Bowen, Journal of Dental Research, Vol. 58/5 (Mai 1979), S. 1493 bis 1501, insbesondere 1495 bis 1498.

Als polymerisierbare Monomere in den erfindungsgemäßen dentalen Füllungsmaterialien sind prinzipiell alle für diesen Zweck vorgeschlagenen und geeigneten Verbindungen einsetzbar. Hier seien insbesondere die bekannten Reaktionsprodukte aus Bisphenolen, insbesondere Bisphenol A, und Glycidylmethacrylat, unter der Abkürzung Bis-GMA bekannt, die verschiedenen Alkandioldimethacrylate wie 1,6-Hexandiolmethacrylat, 1,4-Butandioldimethacrylat, Tri- oder Tetraethylenglykoldimethacrylat, Bis(2-methycroylropyl)-phthalat, -isophthalat oder -terephthalat, Trimethylolpropandi- und trimethacrylat, sowie insbesondere die Reaktionsprodukte aus Diisocyanaten und Hydroxyalkylmethylacrylaten, wie sie beispielsweise in der DE-OS 2 312 559 beschrieben sind, Addukte aus (Di)Isocyanaten und 2,2-Propanbis-[3-(4-Phenoxy)-1,2-hydroxypropan-1-methacrylat] nach der US-PS 3 629 187 sowie insbesondere die Addukte aus Isocyanaten und Methacryloylalkylethern, -alkoxybenzolen bzw. -alkoxycycloalkanen, wie sie in der EP-A-44352 beschrieben sind, genannt.

Selbstverständlich können auch Gemische aus geeigneten Monomeren verwendet werden.

Die mit dem erfindungsgemäßen Füllstoff ausgestatteten dentalen Füllstoff ausgestatteten dentalen Füllungsmaterialien können in zwei Modifikationen vorliegen; entweder als zweiphasige Präparate, von denen die eine Phase einen Polymerisationsinitiator beispielsweise ein Peroxid, und die andere Phase einen Beschleuniger für dieses Peroxid, beispielsweise ein organisches Amin, enthält, wobei das Zusammenbringen beider Phasen unmittelbar vor der Zahnfüllung erfolgt und die Polymerisation in der aufgebohrten, vorzugsweis mit einem Unterfütterungsmaterial versehenen, zu füllenden Kavität eintritt.

Es ist jedoch auch möglich, einphasige Präparate herzustellen, die unter Einwirkung von Licht, beispielsweise UV- oder Laserlicht polymerisieren und dann selbstverständlich einen Photopolymerisationsinitiator und gegebenenfalls auch einen Beschleuniger dafür enthalten.

Entsprechende Photopolymerisationsinitiatoren sind bekannt, vorzugsweise handelt es sich dabei um Carbonylverbindungen wie Benzoin und dessen Derivate, insbesondere Benzoinmethyläther, Benzil und Benzilderivate, beispielsweise 4,4-Oxidibenzil oder andere Dicarbonylverbindungen, z. B. Diacetyl, 2,3-Pentandion oder Metallcarbonyle, Chinone oder deren Derivate. Der Anteil an Photopolymerisationsinitiator beträgt etwa 0,01 bis etwa 5 Gew.-% der Gesamtzusammensetzung.

Diese im Licht härtbaren, d. h. photopolymerisierbaren Präparate enthalten vorzugsweise nocht sogenannte Polymerisationsbeschleuniger. Dies sind Substanzen, die in Gegenwart von Polymerisationsinitiatoren die Polymerisationsreaktion beschleunigen. Bekannte Beschleuniger sind bespielsweise Amine wie p-Toluidin, Dimethyl-p-toluidin, Trialkylamine wie Trihexylamin, Polyamine wie N, N, N', N'-Tetraalkylalkylendiamine, Barbitursäure und Dialkylbarbitursäuren und Sulfimide, vorzugsweise in einer Menge von etwa 0,01 bis etwa 5 Gew.-% der Gesamtzusammensetzung.

Soll das den erfindungsgemäßen Füllstoff mit einem Organosilikon-Gehalt von 5 bis 25% enthaltende Dentalfüllungsmaterial nicht lichthärtbar sein und in zwei bis zur Anwendung voneinander getrennt gehaltenen Phasen vorliegen, so enthält eine dieser Mischungen in der Regel einen Polymerisationsinitiator.

Dies sind zumeist Peroxide die bei der Auslösung der Polymerisation unter Radikalbildung zerfallen. Geeignete Peroxide sind beispielsweise Arylperoxide wie Benzoylperoxid, Cumolhydroperoxid, Harnstoffperoxid, tert.-Butylhydroperoxid oder -perbenzoat und Silylperoxide, vorzugsweise in Mengen von etwa 0,01 bis etwa 0,5 bis 2,5 Gew.-% der Gesamtzusammensetzung.

Enthält die eine Phase des zweiphasigen Mittels einen Polymerisationsinitiator, so wird der anderen Phase zweckmäßigerweise ein Beschleuniger des oben beschriebenen Typs, vorzugsweise ein Amin oder Barbitursäure oder deren Derivate, beispielsweise eine Dialkylbarbitursäure, zugesetzt.

Es ist schließlich zweckmäßig, dentalen Füllungsmaterialien auf Kunststoffbasis UV-Stabilisatoren

zuzusetzen, um das Nachdunkeln während des Alterns der Füllungen zu vermeiden. Ein besonders geeigneter UV-Stabilisator ist 2-Hydroxy-4-methoxybenzophenon. Ein weiteres bevorzugtes Material ist 2-(2'-Hydroxy-5'-methylphenyl)benzotriazol; jedoch ist prinzipiell jedes physiologisch inerte UVabsorbierende Agens für diesen Zweck geeignet. So seien beispielhaft noch Hydrochinon, p-Benzochinon, -p-Butylhydroxytoluol u. ä. genannt. Die letztere Verbindung kann beispielsweise auch als Antioxidans in der Füllung wirken.

Eine Übersicht über die in dentalen Füllungsmaterialien üblicherweise zum Einsatz gelangenden Substanzen gelangenden Substanzen findet sich in dem bereits erwähnten Artikel von R. L. Bowen im Journal of Dental Research, Vol. 58/5 (Mai 1979, S. 1493 bis 1503, sowie der daran angeschlossenen Ergänzung von J. F. Lann, S. 1504 bis 1506.

Zur Einstellung eines möglichst naturgetreuen Eindrucks der gefüllten Zahnflächen enthalten Composite-Materialien erforderlichenfalls auch einen geringen Anteil an Farbstoffen oder Pigmenten.

Die nachfolgenden Beispiele dienen der Erläuterung der Erfindung.

## Herstellungsbeispiel A

### Herstellung eines hochsilanisierten Füllstoffs mit einer Teilchengröße zwischen 10 und 50 Nanometer

200 g Methacroylpropyltrimethoxysilan, 3 g Essig und 350 g entionisiertes Wasser werden bei Raumtemperatur während 30 Minuten zusammengerührt. Diesem Gemisch werden 2000 g Aceton zugesetzt und dann, stufenweise, 1000 g mikroporöses Silica mit einer mittleren Teilchengröße von 10 bis 50 nm und einer spezifischen Oberfläche von 60 $m^2/g$ zugefügt.

Die erhaltene Paste wird bei 50° C getrocknet und durch ein 325 Mesh-Sieb gedrückt, wobei 1150 g eines feinen weißen Pulvers mit einem pH-Wert von 8 erhalten werden.

## Beispiel 1

| Lichthärtbares Füllungsmaterial | Gew.-Teile |
|---|---|
| Reaktionsprodukt aus Bisphenol A und Glycidylmethacrylat (Bis-GMA) | 25 |
| Methylen-4,4', N, N'-bis-p-toluylencarbamat des 3-Methacroyl-2-hydroxypropoxybenzols | 25 |
| Silanisiertes $SiO_2$ (10% Organosilikon; mittlerer Teilchendurchmesser ~30—50 nm) | 15 |
| Bariumsilikat (2% Organosilikon; mittlerer Teilchendurchmesser ~2—10 Mikrometer) | 170 |
| Pyrogenes $SiO_2$ (Aerosil®) | 1,5 |
| Benzil | 1 |
| Trihexylamin | 1,8 |
| gegebenenfalls Spuren Farbstoff | |

Nach dem Auspolymerisierbaren wurde ein Produkt mit folgenden Eigenschaften erhalten:

| | |
|---|---|
| Diametrale Zugfestigkeit: | 55,5 MPa (8050 psi) |
| Wasserabsorption: | 0,43 $mg/cm^2$ |
| Härte nach Barcol (nach 1 h): | 96—97° |
| Druckfestigkeit: | 179,3 MPa (26 000 psi) |
| Polierbarkeit mit Shofu-Polierer: | gut. |

## Beispiel 2

| Lichthärtbares Füllungsmaterial | Gew.-Teile |
|---|---|
| Bis-GMA | 25 |
| Methylen-4,4',N,N'-biscyclohexylcarbamat des 3-Methacroyl-2-hydroxy-propoxytoluols | 30 |
| Tetraethylenglykoldimethacrylat | 20 |
| 1,6-Hexandioldimethacrylat | 10 |
| Methylmethacrylat | 15 |
| Silanisiertes $SiO_2$ (12% Organosilikon; mittlerer Teilchendurchmesser 10—50 nm) | 57,7 |
| Bariumaluminiumsilikat (2% Organosilikon; mittlerer Teilchendurchmesser <10 Mikrometer) | 563 |
| Pyrogenes $SiO_2$ (z. B. Aerosil®) | 5 |
| 4,4'-Oxidibenzil | 0,3 |
| Dimethyl-p-toluidin | 0,5 |
| gegebenenfalls Spuren Pigment | |

Eigenschaften des gehärteten Produkts:

| | |
|---|---|
| Diametrale Zugfestigkeit: | 56.5 MPa (8190 psi) |
| Wasserabsorption: | 0,366 mg/$cm^2$ |
| Härte nach Barcol (7 Tage/37° C): | 98—99° |
| Druckfestigkeit: | 206.8 MPa (30 000 psi) |
| Polierbarkeit mit Shofu-Polierer: | gut |

## Beispiel 3

| Zweiphasiges Füllungsmaterial | Zusammensetzung A Gew.-Teile | B Gew.-Teile |
|---|---|---|
| 2,2-Bis-[4'-(3"methacroyl-2"-hydroxypropoxy)phenyl] propan (Bis-GMA) | 12 | 12 |
| 2,2-Bis-[4'-(2"-methacroylethoxy)-phenyl] propan (EBA) | 66 | 66 |
| Triethylenglykoldimethacrylat | 22 | 22 |
| Tert.-Butylhydroxyltoluol (BHT) | 0,03 | 0,15 |
| UV-Absorber | 0,6 | 0,6 |
| Benzoylperoxid | | 2,0 |
| N,N-(2-Hydroxyethyl)-p-toluidin | 3,6 | |
| Eisenoxid-Pigment | 0,02 | |
| Füllstoff, hergestellt nach Beispiel A | 135 | 135 |

Nach der Aushärtung bei 23° C wurde ein Produkt mit folgenden Eigenschaften erhalten:

| | |
|---|---|
| Härte (Barcol): | 96 |
| Diametrale Zugfestigkeit: | 34,5 MPa (5 000 psi) |
| Polierfähigkeit: | ausgezeichnet |

Beispiel 4

| Zweiphasiges Füllungsmaterial | Zusammensetzung A Gew.-Teile | B Gew.-Teile |
|---|---|---|
| Bis-GMA | 12 | |
| EBA | 66 | |
| Triethylenglykoldimethacrylat | 22 | |
| Hexandioldimethacrylat | | 28 |
| Methylen-4,4', N, N'-biscyclohexylcarbamat von 3-Methacroyl-2-hydroxypropoxybenzol (Urethanmethacrylat) | | 72 |
| UV-Absorber | 0,6 | 0,6 |
| BHT | 0,03 | 0,15 |
| Dimethyl-p-toluidin | 3,5 | |
| Benzoylperoxid | | 2,2 |
| Glaskeramik-Füllstoff nach DE-AS 2 347 591 (Teilchengröße von 1 bis 10 Mikrometer) | 125 | 125 |
| Füllstoff, hergestellt nach Beispiel A | 80 | 60 |

Eigenschaften des Produktes nach der Polymerisation bei 23° C:

| | |
|---|---|
| Härte (Barcol): | 97 |
| Diametrale Zugfestigkeit: | 39,3 MPa (5 700 psi) |
| Polierbarkeit: | gut |

Beispiel 5

| Zweiphasiges Füllungsmaterial | Zusammensetzung A Gew.-Teile | B Gew.-Teile |
|---|---|---|
| Bis-GMA | 12 | |
| EBA | 66 | |
| Triethylenglykoldimethacrylat | 22 | |
| Hexandioldimethacrylat | 12 | 30 |
| Urethanmethacrylat | 12 | 70 |
| UV-Absorber | 0,6 | 0,6 |
| Diäthyl-p-toluidin | 3,6 | |

Tabelle (Fortsetzung)

| Zweiphasiges Füllungsmaterial | Zusammensetzung A Gew.-Teile | B Gew.-Teile |
|---|---|---|
| BHT | 0,03 | 0,15 |
| Cumolhydroperoxid | | 2,2 |
| Eisenoxid-Pigment | 0,02 | |
| Füllstoff, hergestellt nach Beispiel A | 94 | 94 |
| Corning® Glass 7724 (Quartz); mittlere Teilchengröße 2 Mikrometer; 100% 40 Mikrometer | 750 | 550 |

Eigenschaften des bei 23° C ausgehärteten Polymerisats:

| | |
|---|---|
| Härte (Barcol): | 99 |
| Diametrale Zugfestigkeit: | 9.6 MPa (7 200 psi) |
| Polierbarkeit: | gut |

Beispiel 6

| Zweiphasiges Füllungsmaterial | Zusammensetzung A Gew.-Teile | B Gew.-Teile |
|---|---|---|
| Bis-GMA | 12 | |
| EBA | 66 | |
| Triethylenglykoldimethacrylat | 22 | |
| Hexandioldimethacrylat | | 30 |
| Urethanmethacrylat | | 70 |
| UV-Absorber | 0,6 | 0,6 |
| Diäthylbarbitursäure | 3,6 | |
| BHT | 0,03 | 0,15 |
| Benzoylperoxid | | 2,2 |
| Farbstoff | 0,02 | |
| Lanthansilikat-Glas-Füllstoff (unter 10 Mikron; mittlerer Teilchendurchmesser 0,5 bis 1 Mikrometer) | 350 | 300 |
| Füllstoff, hergestellt nach Beispiel A | 30 | 30 |

Eigenschaften des nach der Aushärtung bei 13° C erhaltenen Produkts:

| | |
|---|---|
| Härte (Barcol): | 98 |
| Diametrale Zugfestigkeit: | 42.7 MPa (6 200psi) |
| Polierbarkeit: | gut |

Beispiel 7

| Zweiphasiges Füllungsmaterial | Zusammensetzung A Gew.-Teile | B Gew.-Teile |
|---|---|---|
| Bis-GMA | 10 | 12 |
| EBA | 60 | 66 |
| Triethylenglykoldimethacrylat | 22 | 22 |
| UV-Absorber | 0,6 | 0,6 |
| Diäthylbarbitursäure | 3,6 | |
| BHT | 0,03 | 0,15 |
| Benzoylperoxid | | 2,2 |
| Farbstoff | 0,02 | |
| Corning® 7740 Borosilikat-Glas (unter 40 Mikrometer; mittlerer Teilchendurchmesser ~2 Mikrometer) | 200 | 200 |
| Füllstoff, hergestellt nach Beispiel A | 50 | 45 |

Nach der Aushärtung bei 23° C wurde ein Produkt mit folgenden Eigenschaften erhalten:

| | |
|---|---|
| Härte (Barcol): | 97 |
| Diametrale Zugfestigkeit: | 38,6 MPa (5 600 psi) |
| Polierbarkeit: | gut |

**Patentansprüche**

1. Dentales Füllungsmaterial auf Basis polymerisierbarer Verbindungen und anorganischer Füllstoffe, dadurch gekennzeichnet, daß es einen silanisierten Füllstoff, insbesondere poröses Siliciumdioxid, mit einem mittleren Teilchendurchmesser von 5 bis 300, insbesondere 10 und 300 Nanometer, mit einem Organosilikon-Gehalt von 5 bis 25, vorzugsweise 10 bis 20%, bezogen auf das Gesamtgewicht des Füllstoffs, enthält.

2. Dentales Füllungsmaterial nach Anspruch 1, dadurch gekennzeichnet, daß der Füllstoff aus einem Gemisch aus

a) 8 bis 60, vorzugsweise 12 bis 60, Gew.-% mindestens eines silanisierten Füllstoffs mit einem mittleren Teilchendurchmesser von 5 bis 300 Nanometer und einem Organosilikon-Gehalt zwischen 5 bis 25% (bezogen auf den Füllstoff),

und

b) 40 bis 92 Gew.-% mindestens eines Füllstoffs mit einem mittleren Teilchendurchmesser von mindestens 0,5 Mikrometer besteht.

3. Dentales Füllungsmaterial nach Anspruch 2, dadurch gekennzeichnet, daß der Füllstoff b) mit einem mittleren Teilchendurchmesser von mindestens 0,5 Mikrometer ein nichtporöser Füllstoff ist.

4. Dentales Füllungsmittel nach Anspruch 2 und 3, dadurch gekennzeichnet, daß der Füllstoff b) silanisiert ist.

5. Dentales Füllungsmaterial nach einem oder mehreren der vorhergehenden Ansprüche, dadurch gekennzeichnet, daß der Füllstoff a) einen mittleren Teilchendurchmesser zwischen 10 und 100 Nanometer aufweist.

6. Dentales Füllungsmaterial nach einem oder mehreren der vorhergehenden Ansprüche, dadurch gekennzeichnet, daß der silanisierte Füllstoff a) einen Organosilikon-Gehalt von 10 bis 20% (bezogen auf den Füllstoff) aufweist.

**Claims**

1. Dental filling material containing polymerizable compounds and anorganic fillers, characterized in that it contains a silanized filler, preferably poreous silica, with an average particle diameter from 5 to 300, preferably 10 and 300 nanometers, having an organosilicon-content from 5 to 25, preferably 10 to 20%, related to the total weight of the filler.

2. Dental filling material according to claim 1, characterized in that the filler ist composed of an mixture of

a) 8 to 60, preferably 12 to 60% by weight of at least one silanized filler with an average particle diameter from 5 to 300 nanometers having an organosilicon content from 5 to 25% (related to the filler),

and

b) 40 to 92% by weight of at least one filler with an average partile diameter of at least 0,5 micrometers.

3. Dental filling material according to claim 2, characterized in that the filler b) with an average particle diameter of a least 0,5 micrometers is a non-poreous filler.

4. Dental filling material according to claims 2 and 3, characterized in that the filler b) is silanized.

5. Dental filling material according to one or more of the preceding claims, characterized in that the filler a) has an average particle diameter from 10 to 100 nanometers.

6. Dental filling material according to one or more of the preceding claims, characterized in that the silanized filler a) has an organosilicon content from 10 to 20% (related to the filler).

**Revendications**

1. Matière dentaire d'obturation contenant des compositions polymérisables et des charges inorganiques caractérisée en ce contient une charge silanisée, particulièrement du silice poreuse avec un diamètre moyen de particles de 5 à 300, particulièrement 10 et 300 nanomètres ayant une teneur d'organo-silicone de 5 à 25, particulièrement 10 à 20%, en relation avec le poids total de la charge.

2. Matière dentaire d'obturation selon revendication 1, caractérisée en ce que la charge se compose d'un mélange de

a) 8 à 60, particulièrement 12 à 60% en poids d'au moins une charge silanisée avec un diamètre moyen de particles de 5 à 300 nanomètres et une teneur d'organo-silicone entre 5 à 25% (en relation avec la charge)

et

b) 40 à 92% en poids d'au moins une charge avec un diamètre moyen de particles d'au moins 0,5 micromètres.

3. Matière dentaire d'obturation selon revendication 2 caractérisée en ce que la charge b) avec un diamètre moyen de particles d'au moins 0,5 micromètres est une charge non-poreuse.

4. Matière dentaire d'obturation selon revendication 2 et 3 caractérisée en ce que la charge b) est silanisée.

5. Matière dentaire d'obturation selon une ou plusieurs des revendications précédentes caractérisée en ce que la charge a) a un diamètre moyen de particles de 10 à 100 nanomètres.

6. Matière dentaire d'obturation selon une ou plusieurs des revendications précédentes caractérisée en ce que la charge silanisée a) a une teneur d'organo-silicone de 10 à 20% (en relation avec la charge).